Europäisches Patentamt

European Patent Office

Office européen des brevets

(11) Publication number: **0 404 300 A2**

## EUROPEAN PATENT APPLICATION

(21) Application number: 90303498.1

(22) Date of filing: 02.04.90

(51) Int. Cl.5: **C12P 7/64, C12P 9/00, C12N 1/20, A61K 37/22**

The microorganism(s) has (have) been deposited with Fermentation Research Institute Agency Industrial Science and Technology under numbers 1623 - 1624 - 1625 - 1626.

(30) Priority: 22.06.89 JP 158161/89

(43) Date of publication of application:
27.12.90 Bulletin 90/52

(84) Designated Contracting States:
AT BE CH DE DK ES FR GB GR IT LI LU NL SE

(71) Applicant: TOSOH CORPORATION
4560, Oaza-Tonda Shinnanyo-shi
Yamaguchi 746(JP)

Applicant: SAGAMI CHEMICAL RESEARCH CENTER
4-5, Marunouchi 1-chome
Chiyoda-ku Tokyo 100(JP)

(72) Inventor: Yazawa, Kazunaga
D1-501 Gurin Haitsu 571, Unomori
Sagamihara-shi, Kanagawa(JP)
Inventor: Kimura, Shuichi
1-33-5, Tsurugaya
Sendai-shi, Miyagi(JP)
Inventor: Kanai, Akira
14-3 Komanba, 2 chome
Meguro-ku, Tokyo(JP)
Inventor: Kondo, Kiyoshi
5-16-4 Chuorinkan
Yamato-shi, Kanagawa(JP)
Inventor: Takahashi, Keiko
2-33-29, Oinezuka Kounosu-shi
Saitama(JP)
Inventor: Ishikawa, Chikako
302 Sofutotaun, Harajuku, 3-13-8 Sendagaya
Shibuya-ku, Tokyo(JP)

(74) Representative: Kearney, Kevin David Nicholas et al
KILBURN & STRODE 30 John Street
London, WC1N 2DD(GB)

(54) Process for production of eicosapentaenoic acid-containing phospholipid and use of said lipid.

(57) A process for the production of an eicosapentaenoic acid (EPA)-containing phospholipid, comprising the steps of preparing cells of microorganism capable of producing EPA, extracting the cells with a solvent which can solubilize the EPA-containing phospholipid, removing the solvent to obtain a residue containing the EPA-containing phospholipid mixing the residue with a solvent which substantially cannot solubilize the EPA-containing phospholipid recovering the solvent insoluble fraction, and purify the fraction by column chromatography; and a lipid metabolism modifying composition comprising an EPA-containing phospholipid.

# PROCESS FOR PRODUCTION OF EICOSAPENTAENOIC ACID-CONTAINING PHOSPHOLIPID AND USE OF SAID LIPID

The present invention relates to a process for the production of eicosapentaenoic acid (abbreviated as EPA thereinafter)-containing phospholipid preferably from marine microorganisms, and the use of the lipid, especially as a lipid metabolism modifier. The present phospholipid is also useful in the foodstuff, cosmetics, pharmaceuticals, agriculture, fishing, and chemical industries.

Phospholipids are present in various organisms including higher animals and plants, and are responsible for important functions as a component of the cell membrane. The phospholipids industrially produced are soybean phospholipid and yolk phospholipid. These phospholipids are natural surfactants, and their emulsifying action, dispersing action, wetting action, etc. make them useful in the fields of foodstuff, cosmetics, pharmaceuticals and the like. Nevertheless, the soybean phospholipid and yolk phospholipid, as well as other phospholipids of higher animal or plant origin, comprise fatty acids having mainly 16 to 18 carbon atoms, and phospholipids comprising EPA, which has 20 carbon atoms, have not yet been industrially isolated. A process for isolating EPA-containing phospholipid from chlorella (Japanese Unexamined Patent Publication, KOKAI, No. 62-56497) has been proposed, but the growth rate is too low for it to be used as an industrial material from which phospholipid is isolated.

The abnormality of the lipid metabolism is partially responsible for "adult" diseases such as arteriosclerosis, thrombosis, hyperlipemia, obesity, hypertension, and diabetes, and although various medicaments have been proposed for the prophylaxis and treatment of these diseases, they do not provide satisfactory results from the viewpoints of pharmacological effectiveness and side-effects, and therefore, there is an urgent need for more effective and safer pharmaceuticals. Recently, EPA has attracted attention, due to its high safety factor, and to date, an EPA in the form of an ethyl ester or triglyceride has been developed. Nevertheless, an EPA linked to phospholipid immediately provides an effect, at a dose of one tenth of the ethyl ester or triglyceride form, and accordingly, a chemical synthesis of EPA-linked phospholipid has been attempted (Japanese Unexamined Patent Publication, KOKAI, Nos. 63-264422 and 58-38215).

This chemical synthesis, however, is disadvantageous in that it is difficult to prevent an incorporation of undesirable impurities, and the cost of the synthesis and purification are high due to a low reaction yield, and thus it is not practically marketable.

Accordingly, the present invention provides a process for the production of an EPA-containing phospholipid, which process can be carried out industrially and efficiently in a short time and with little trouble, and a lipid metabolism modifier comprising EPA-containing phospholipid.

More specifically, the present invention provides a process for the production of an eicosapentaenoic acid (EPA)-containing phospholipid, comprising the steps of:

preparing cells of microorganism capable of producing EPA;

extracting the cells with a solvent which can solubilize the EPA-containing phospholipid;

removing the solvent to obtain a residue containing the EPA-containing phospholipid;

mixing the residue with a solvent which substantially cannot solubilize the EPA-containing phospholipid;

recovering the solvent-insoluble fraction; and

purify the fraction by column chromatography.

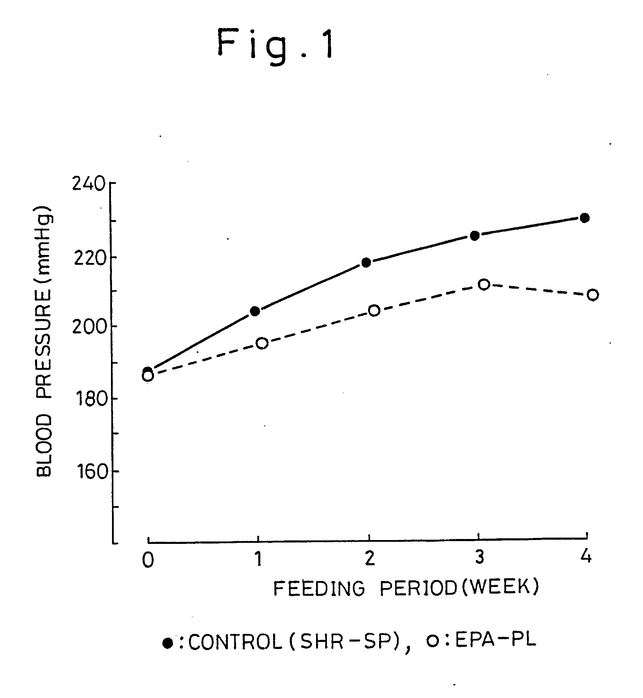

Figure 1 represents the relationship between feeds used in Example 1 and changes of blood pressure.

The present inventors obtained the following finding concerning a lipid produced by a microorganism capable of producing EPA: (1) a total lipid content of a microorganism capable of producing EPA is as high as 6 to 12%, on the basis of dry matter, and at least 90% of the total lipid is a phospholipid; (2) in the fatty acid composition of the total lipid, the content of EPA is as high as 24 to 40%, and only a fatty acid having a high degree of unsaturation is EPA; (3) a high purity phospholipid can be easily obtained by solvent-fractionation of the total lipid; and (4) when total lipid is fractionated into a phospholipid, free fatty acids, quinones and other components, by solvent-fractionation and silica gel column chromatography, the ratio of EPA to the total of fatty acids in the phospholipid is at least 30%.

This means that microorganisms capable of producing EPA are a preferable source of an EPA-containing phospholipid. Moreover, the present microorganisms capable of producing EPA have remarkably higher growth rate than that of many other micro organisms, and therefore, a large amount of microbial cells can be obtained in a very short time. In addition an administration of a EPA-containing lipid or cells of a microorganism capable of producing EPA provides an efficient decrease of lipid in the blood and liver, as well as the weight of adipose tissue in animals.

In the present invention, any microorganisms capable of producing EPA may be used. These microorganisms belong to, for example, the genus Pseudomonas, Alteromonas, Shewanella, Deleya, Alteromarinus, Pasteurella, or Vibrio. The properties of these microorganisms are described in detail in E.P. Application No. 87311372.4 (Publication No. 0273708); and Japanese patent application (Unexamined Publication, KOKAI) Nos. 62-49929 (63-216488), 63-49930 (63-216489), 62-49931 (63-216490), 62-156693 (1-2587) and 62-273200 (2-23877).

Microorganisms capable of producing EPA and belonging to the genus Pseudomonas include Pseudomonas putrefaciens SCRC-2181, SCRC-2201, SCRC-2271, SCRC-2341, SCRC-2451, SCRC-2642, SCRC-2792, SCRC-2878, SCRC-3011, and SCRC-3022. Micoorganisms capable of producing EPA and belonging to the genus Alteromonas include Alteromonas putrefaciens SCRC-2871, Alteromonas putrefaciens subspecies sagamifaciens SCRC-1162, Alteromonas lumensas SCRC-6444, and Alteromonas xylosus SCRC-2517. Microorganisms capable of producing EPA and belonging to the genus Shewanella include Shewanella putrefaciens SCRC-2874.

The above-mentioned microorganisms were deposited with the Fermentation Research Institute Agency of

Industrial Science and Technology, Ministry of International Trade and Industry, 1-1-3 Yatabe-machi Higashi, Tsukuba-shi, Ibaraki-ken, Japan under FERM P-Numbers, and transferred to international deposition under the Deposit of Microorganisms for the Purposes of Patent Procedure (Budapest Treaty) under FERM BP-Numbers as follows:

Pseudomonas putrefaciens SCRC-2878
December 26, 1986 FERM P-9114
December 17, 1987 FERM BP-1623
Alteromonas putrefaciens SCRC-2871
January 28, 1987 FERM P-9158
December 17, 1987 FERM BP-1624
Shewanella putrefaciens SCRC-2874
January 28, 1987 FERM P-9159
December 17, 1987 FERM BP-1625
Alteromonas putrefaciens subspecies sagamifaciens SCRC-1162
February 20, 1987 FERM P-9210
December 17, 1987 FERM BP-1626.

The above-mentioned strains for the present invention may be converted to strains having an eicosapentaenoic acid productivity higher than the original strain, by conventional mutation and selection procedures.

The microorganisms used in the present invention can be preserved by a conventional method, such as on an agar slant medium or by lyophilization. As the agar for culturing the microorganism, any medium in which the microorganism used can grow and produce eicosapentaenoic acid can be used. The medium contains, as a nitrogen source, for example, yeast extract, peptone or meat extract, or a combination thereof. Moreover, the medium may contain, as a carbon source, various kinds of sugar such as glucose, fructose, maltose, and sucrose; organic acids, such as intermediates of the TCA cycle such as citrate, fumarate and pyruvate, other organic acids such as maleate and acetate; and amino acids such as aspartate and glutamate. The medium preferably contains sodium chloride, or sea water or artificial sea water. Note, Alteromonas putrefaciens, subspecies sagamifaciens require sodium chloride.

In a preferred embodiment of the present invention, a medium containing 1.0% peptone, 0.5% yeast-extract, and 1.5% NaCl in tap water (pH 7.0) is used.

The microorganisms of the present invention can be cultured in a liquid medium or on a solid medium. The pH value is 6 to 9, preferably 7 to 8. To obtain a large amount of the cells the microorganism is cultured in a liquid medium by stationary culture, or more preferably, by a shaking culture or aeration-agitation culture, under aerobic conditions. The microorganism is cultured at any temperature at which the microorganism can grow and produce eicosapentaenoic acid. The temperature is preferably 5°C to 30°C, and more preferably, 15°C to 25°C. Culturing is carried out for a period sufficient to produce eicosapentaenoic acid in an amount enough to enable the recovery of EPA-containing phospholipid, preferably for 8 to 48 hours.

The cultured cells are recovered by a conventional procedure such as centrifugation, and dried by a conventional procedure such as lyophilization, spray-dry or drum-dry to obtain a microbial cell powder.

Next, to obtain a crude phospholipid fraction, the dried cells are extracted with a solvent, preferably an organic solvent or a mixture thereof which can solubilize phospholipid, such as chloroform/methanol, n-hexane/ethanol/water, chloroform/hexane, chloroform/ether, chloroform/ethanol, or the like.

The extract is then evaporated to remove the solvent, and the resulting residue is subjected to organic solvent-fractionation. For the fractionation, for example, acetone, acetic acid, chloroform, or ether, such as ethyl ether, is used. The solvent for the fractionation may contain inorganic salts such as magnesium chloride, calcium chloride or the like. The fractionation is usually carried out by mixing the extract residue with a fractionation solvent which cannot solubilize the phospholipid, such as acetone at a temperature -80°C and 40°C, for example at room temperature, and separating an insoluble fraction containing the phospholipid from the solvent.

If necessary, the phospholipid-containing fraction obtained as described above is further purified by column chromatography, to obtain a highly purified phospholipid composition. The column chromatography used is, for example, hydrophobic chromatography, chromatography using a silica-series carrier, chromatography using a polymer gel such as Toyopearl (Tosoh, Japan), and chromatography using octadecyl-linked silica and the like.

The thus obtained phospholipid composition may be analyzed to determine the phospholipid components thereof, by thin layer chromatography using as a solvent system, for example, chloroform/methanol/water. According to the present invention, a phospholipid composition mainly comprises phosphatidylethanolamine and phosphatidylglycerol. The phospholipid composition also may be analyzed to determine the fatty acid content thereof, by saponifying the phospholipid, methylating the saponification product, and analyzing the methylated fatty acids by gas chromatography. For example, the present phospholipid composition contains EPA, oleic acid, palmitoleic acid, palmitic acid, and myristic acid, and trace amounts of other fatty acids.

The phospholipid prepared from a microorganism capable of producing EPA, and cells of the microorganism, can be used as a lipid metabolism modifier. The dose of the phospholipid is approximately 1 mg to 1 g of EPA-linked phospholipid/kg body weight/day. Where microbial cells per se are administered, an amount of about 20 fold of the dose of the phospholipid provides approximately the same ffect as the phospholipid. Any formulation can be used, but preferable formulations are, for example, capsule, granule, pill, suspension, emulsion, powder, tablet, syrup, and an injectable liquid. These pharmaceutical preparations may contain pharmaceutically or biologically acceptable additives.

The present lipid metabolism modifier can be used not only for pharmaceuticals for the prophylaxis and treatment of human diseases, and of animals such as domestic animals and pets, but also as a component of a health food and of a feed such as chicken feed for the production of low-cholesterol eggs.

Next, an example of a composition of feed is shown.

TABLE 1

| Components | Content |
|---|---|
| Casein | 20% |
| EPA-PL* or butter | 20% |
| Cholesterol | 0.5% |
| Sodium cholate | 0.25% |
| Mineral mixture | 4% |
| Vitamin mixture | 2% |
| Corn starch | 53.25% |

* EPA-PL: EPA-containing phospholipid.

The present invention will now be further illustrated by, but is by no means limited to, the following examples.

Example 1

Alteromonas putrefaciens SCRC-2871 (FERM BP-1624), which can produce EPA, was cultured in a medium containing peptone 1%, yeast extract 0.5%, and NaCl 1.5% (pH 7) at 25°C for 18 hours, under an aerobic condition by a 10l-jar fermenter, and the cultured broth was centrifuged to collect cells, which were then washed with water and dried by lyophilization to obtain a cell powder. Then 100 g of the cell powder was extracted with 5 l of a mixture of chloroform/methanol (2:1), and the extract was evaporated to obtain 8 g of a lipid extract.

To the lipid was added 400 ml of acetone, the mixture was stirred while cooling, and then filtered to recover an acetone-insoluble fraction. The acetone-insoluble fraction was dried to obtain about 7 g of a phospholipid composition (abbreviated as EPA-PL). The phospholipid component of the EPA-PL was determined by thin layer chromatography, and the fatty acid composition was determined as described above by gas chromatography. As a result, the EPA-PL comprised mainly phosphatidylethanolamine and phosphatidyl-glycerol; and the fatty acid components were EPA, oleic acid, palmitic acid, palmitoleic acid, and myristic acid, and trace amounts of other fatty acids.

The EPA-PL was used in a feed shown in Table 1. As a control, a feed wherein the EPA-PL was replaced by 20% butter was prepared. The EPA-PL feed or the butter feed (control) was administered to SHR-SP rats (stroke-prone spontaneously hypertensive rat, male, body weight 200 g) in an amount of 5 g/day, and a normal feed (SP feed, Funabashi) was also given in an amount of 13 g/day.

The animals were fed for 4 weeks, and during this period, the tail artery pressure was measured at one-week intervals.

After feeding the animals for 4 weeks, they were dissected, and weight of the adipose tissue, the amount of triglyceride in the liver, and the plasma lipid were measured. The liver glyceride was measured by the acetylacetone method, after an extraction of the total lipid with a chloroform/methanol (2:1) mixture. The plasma total cholesterol was measured by Zurkowski method; and VLDL (very low density lipoprotein) and chylomicron were measured by fractionating to each lipoprotein by ultracentrifugation and then measuring the cholesterol content therein by the above-mentioned method. The plasma triglyceride and phospholipid were measured by the acetylacetone method and phosphorus permanganate method, respectively.

The thus-obtained results are shown in Figure 1 and Tables 2 to 4. Note, in Tables 2 to 4, the number in parantheses shows the rate (%) of a decrease of a value calculated by taking the control as 100%.

TABLE 2

| Decrease of weight of the adipose tissue. | | |
|---|---|---|
| | EPA-PL feed | Control feed |
| Perirenal adipose tissue | 2.21 ± 0.51 g (-38.3%) | 3.58 ± 1.05 g |
| Paraepididymal adipose tissue | 3.09 ± 0.29 g (-26.4%) | 4.20 ± 0.38 g |

TABLE 3

| Decrease of triglyceride in the liver. | | |
|---|---|---|
| | EPA-PL feed | Control feed |
| Liver triglyceride | 7.7 ± 2.2 mg/g (-35.3%) | 11.9 ± 4.9 mg/g |

EP 0 404 300 A2

TABLE 4

| Decrease of plasma lipid. | | |
|---|---|---|
| | EPA-PL feed | Control feed |
| Plasma | mg/dl | mg/dl |
| total cholesterol | 44.3 ± 3.2 (-30.6%) | 63.8 ± 7.4 |
| VLDL + chylomicron Cholesterol | 11.5 ± 2.9 (-44.7%) | 20.8 ± 7.4 |
| Triglyceride | 48.3 ± 10.6 (-20.0%) | 60.4 ± 8.9 |
| Phospholipid | 101.7 ± 10.8 (-14.7%) | 119.2 ± 5.8 |

Example 2

A marine microorganism Shewanella putrefaciens SCRC-2874 (FERM BP-1625) was cultured in the medium containing meat extract 1%, peptone 1% and NaCl 0.5% (pH 7.0), and the cultured broth was centrifuged to obtain cells, which were then washed and dried to obtain a microbial cell powder, as described in Example 1. Next, 100 g of the cell powder were extracted with a solvent mixture of n-hexane/ethanol/water (2:0.9:0.1), and the extract was evaporated to obtain 12 g of a total lipid. To the total lipid was added 500 ml of acetone, and the mixture was stirred while cooling, and separated into an acetone-soluble fraction containing fatty acids and quinone, and an acetone-insoluble fraction containing the phospholipid. The thus-obtained acetone-insoluble fraction was dissolved in butanol, and fractionated by silica gel column chromatography, using as an eluate butanol/acetic acid/water, as described in Example 1. Each fraction was tested for the presence of the phospholipid, and fractions containing the phospholipid were combined to obtain 10.5 g of a phospholipid composition. The composition contained, as main components, phosphatidylglycerol and phosphatidylethanolamine; and the fatty acid components comprised EPA, oleic acid, palmitic acid, palmitoleic acid, and myristic acid, and trace amounts of other fatty acids.

As seen from the above, according to the present invention, there is provided a process for a production of an EPA-containing phospholipid, which process is efficient and makes it possible to isolate and purify the desired phospholipid in a short time, and a safe lipid metabolism modifier.

Example 3

A marine microorganism Pseudomonas putrefaciens SCRC-2878 (FERM BP-1623) was cultured in the medium containing meat extract 1%, peptone 1% and NaCl 0.5% (pH 7), and the cultured broth was centrifuged to obtain cells, which were then washed and dried to obtain a microbial cell powder, as described in Example 1. Next, 100 g of the cell powder were extracted with a solvent mixture of chloroform/methanol (2:1), and the extract was evaporated to obtain 8 g of a total lipid. To the total lipid was added 400 ml of acetone, and the mixture was stirred while cooling, and separated into an acetone-soluble fraction containing fatty acids and quinone, and an acetone-insoluble fraction containing the phospholipid. The thus-obtained acetone-insoluble fraction was dissolved in chloroform, and fractionated by silica gel column chromatography, using as eluates chloroform, chloroform/methanol ( 2 : 1 ), chloroform/methanol (1:1), and methanol in this order. Each fraction was tested for the presence of the phospholipid, and fractions containing the phospholipid were combined to obtain about 7 g of a phospholipid composition. The composition contained, as main components, phosphatidyl glycerol and phosphatidylethanolamine as determined by TLC; and the fatty acid components comprised EPA, oleic acid, palmitic acid, palmitoleic acid, and myristic acid, and trace amounts of other fatty acids.

**Claims**

6

1. A process for a production of an eicosapentaenoic acid (EPA)-containing phospholipid, comprising the steps:

of preparing cells of a microorganism capable of producing EPA;

extracting the cells with a solvent which can solubilize the EPA-containing phospholipid;

removing the solvent to obtain a residue containing the EPA-containing phospholipid;

mixing the residue with a solvent which substantially cannot solubilize the EPA-containing phospholipid;

recovering the solvent-insoluble fraction; and

purify the fraction by column chromatography.

2. A process as claimed in Claim 1, characterized in that the microorganism capable of producing EPA is selected from the group consisting of the genera Pseudomonas, Alteromonas, Shewanella, Deleya, Alteromarinous, Pasteurella and Vibrio.

3. A process as claimed in Claim 2, characterized in that the microorganism of the genus Pseudomonas is selected from the group consisting of Pseudomonas putrefaciens SCRC-2181, SCRC-2201, SCRC-2271, SCRC-2341, SCRC-2451, SCRC-2642, SCRC-2792, SCRC-2878, SCRC-3011, and SCRC-3022.

4. A process as claimed in Claim 2, characterized in that the microorganism of the genus Alteromonas is selected from the group consisting of Alteromonas putrefaciens SCRC-2871, Alteromonas putrefaciens subspecies sagamifaciens SCRC-1162, Alteromonas lumensas SCRC-6444, and Alteromonas xylosus SCRC-2517.

5. A process as claimed in Claim 2, characterized in that the microorganism of the genus Shewanella is Shewanella putrefaciens SCRC-2874.

6. A process as claimed in any one of Claims 1 to 5 characterized in that the EPA-containing phospholipid comprises mainly phosphatidylglycerol and phosphatidylethanolamine.

7. A process as claimed in any one of Claims 1 to 6 characterized in that the solvent which can solubilize a phospholipid is selected from the group consisting of chloroform/methanol mixtures, n-hexane/ethanol/water mixtures, chloroform/hexane mixtures, chloroform/ether mixtures, and chloroform/ethanol mixtures.

8. A process as claimed in any one of Claims 1 to 7 characterized in that the solvent whch cannot solubilize a phospholipid is acetone.

9. A lipid metabolism modifying composition comprising an EPA-containing phospholipid.

10. A composition as claimed in Claim 9, characterized in that the phospholipid comprises phosphatidylethanolamine and phosphatidylglycerol.

11. A lipid metabolism modifying composition comprising cells of a microorganism capable of producing EPA.

12. A microorganism capable of producing EPA characterised in that the total lipid content of the microorganism is in the range 6 to 12% on the basis of dry matter, and at least 90% of the total lipid is a phospholipid, the content of EPA in the fatty acid component of the total lipid is in the range 24 to 40% and the only fatty acid present which has a high degree of unsaturation is EPA.

13. A lipid produced by a microorganism capable of producing EPA characterised in that the total lipid content of the microorganism is in the range 6 to 12% on the basis of dry matter, and at least 90% of the total lipid is a phospholipid, the content of EPA in the fatty acid component of the total lipid is in the range 24 to 40% and the only fatty acid present which has a high degree of unsaturation is EPA.

14. The use of cells of a microorganism capable of producing EPA as a lipid metabolism modifying agent in the preparation of a pharmaceutical composition for therapeutic use.

# Fig.1

●:CONTROL(SHR-SP), ○:EPA-PL